# EUROPEAN PATENT APPLICATION

(11) **EP 4 279 498 A1**
(43) Date of publication of application: **22.11.2023**
(21) Application number: 22739741.1
(22) Date of filing: 14.01.2022
(51) Int. Cl.: C07K 7/08, C07K 19/00, C07K 16/00, G01N 33/68, G01N 33/58, G01N 33/53

(54) **SWITCHING PEPTIDE, IMMUNOASSAY DEVICE COMPRISING SAME, AND IMMUNOASSAY METHOD USING SAME**

(30) Priority: 14.01.2021 KR 20210005052
(71) Applicant: Optolane Technologies Inc., Bundang-gu Seongnam-si, Gyeonggi-do 13494 (KR)
(72) Inventor: PYUN, Jae-Chul, Seoul 06521 (KR)
(74) Representative: Schiweck Weinzierl Koch Patentanwälte Partnerschaft mbB
(86) International application number: PCT/KR2022/000693
(87) International publication number: WO 2022/154548

(57) **Abstract**

Disclosed is a switching peptide that can be applied to an optical one-step immunoassay method. The switching peptide comprises: a peptide compound capable of reversibly binding to a binding antibody; and a fluorescent marker bound to the peptide compound.

## Description

### [Technical Field]

The present disclosure relates to a switching peptide that may be used to analyze a target antigen substance using an immune response, an immunoassay device comprising the same, and an immunoassay method using the same.

### [Background Art]

Immunoassay has been used for bio-testing such as various immunodiagnostic tests or environmental monitoring in the field of biotechnology. The immunoassay has the advantage of being able to quantitatively analyze a specific target substance by using the specificity of an antigen-antibody reaction and having higher sensitivity than other analysis methods.

The immunoassay may be used for various diagnoses such as diagnosing cancer markers, infectious diseases, thyroid function, anemia, allergy, pregnancy, drug abuse, and gout. In order to use the immunoassay for diagnosis or analysis of various types of antigens, it is necessary to prepare antibodies having specific reactivity to each of the antigens.

However, such a method has limitations in detecting modernized diseases that have rapidly been diversified recently, or viruses having multiple mutants. In particular, in the case of the virus, it is often impossible to find an antibody having a specific binding property with the virus.

In addition, when the antigen-antibody reaction is used, an additional step of binding the antibody to a label may be required. If the binding property between the label and the antibody is poor, the accuracy or reliability of immunoassay analysis may be deteriorated.

Due to the aforementioned problems, there is also a problem of developing various types of labels capable of binding to various antibodies, respectively.

### [Disclosure]

### [Technical Problem]

One object of the present disclosure is to provide a switching peptide that may be applied to an optical one-step immunoassay method, comprising a peptide compound capable of selectively and reversibly binding to a Fab region of a binding antibody and a fluorescent label capable of emitting fluorescence.

Another object of the present disclosure is to provide an immunoassay device comprising the switching peptide.

Still another object of the present disclosure is to provide an immunoassay method using the immunoassay device.

### [Technical Solution]

A switching peptide according to an embodiment of the present disclosure comprises: a peptide compound capable of reversibly binding to a binding antibody; and a fluorescent label bound to the peptide compound.

In one embodiment, the peptide compound may be selectively and reversibly bound to a fragment antigen-binding (Fab) region of the binding antibody.

In one embodiment, the peptide compound may have an amino acid sequence capable of specifically and reversibly binding to one or more of first to fourth framework regions (FR1, FR2, FR3, and FR4) of a light chain or heavy chain of the binding antibody.

In one embodiment, the peptide compound may comprise one or more selected from the group consisting of a first peptide compound having a first amino acid sequence having homology to an amino acid sequence of the second light chain variable framework region (VL-FR2); a second peptide compound having a second amino acid sequence having homology to an amino acid sequence of the third or fourth light chain variable framework region (VL-FR3, VL-FR4) ; a third peptide compound having a third amino acid sequence having homology to an amino acid sequence of the second heavy chain variable framework region (VH-FR2); and a fourth peptide compound having a fourth amino acid sequence having homology to an amino acid sequence of the third or fourth heavy chain variable framework region (VH-FR3, VH-FR4).

In one embodiment, the peptide compound may comprise 14 to 20 amino acids.

In one embodiment, the peptide compound may have a molecular weight of 1,650 to 2,500 Da.

In one embodiment, the fluorescent label may comprise one or more selected form the group consisting of rhodamine and its derivatives, fluorescein and its derivatives, coumarin and its derivatives, acridine and its derivatives, pyrene and its derivatives, erythrosine and its derivatives, eosin and its derivatives, 4-acetamido-4'-isothiocyanatostilbene-2,2'disulfonic acid, fluorescein isothiocyanate (FITC), oregon green, alex fluoro, carboxyfluorescein (FAM), 6-carboxy-4',5'-dichloro-2',7'-dimethoxyfluorescein (JOE), carboxy-X-rhodamine (ROX), 6-carboxy-2',4,4',5',7,7'-hexachlorofluorescein (HEX), Texas Red (sulforhodamine 101 acid chloride), 6-carboxy-2',4,7',7-tetrachlorofluorescein (TET), tetramethylrhodamine-isothiocyanate (TRITC), carboxytetramethylrhodamine (TAMRA), cyanine-based dyes, and thiadicarbocyanine dyes.

An immunoassay device according to an embodiment of the present disclosure includes: a substrate having a reaction space capable of accommodating a detection sample solution; a binding antibody located inside the reaction space and immobilized on the substrate; a switching peptide comprising a peptide compound reversibly bound to a Fab region of the binding antibody and a fluorescent label bound to the peptide compound; and a quenching material disposed adjacent to the fluorescent label to quench fluorescence from the fluorescent label.

An immunoassay device according to another embodiment of the present disclosure includes: a substrate having a reaction space capable of accommodating a detection sample solution; a support located inside the reaction space; a binding antibody bound to the support; a switching peptide comprising a peptide compound reversibly bound to a Fab region of the binding antibody and a fluorescent label bound to the peptide compound; and a quenching material disposed adjacent to the fluorescent label to quench fluorescence from the fluorescent label.

In one embodiment, the quenching material may be bound to the Fc region of the binding antibody or the substrate.

In one embodiment, the quenching material may be bound to the Fc region of the binding antibody or the support.

In one embodiment, the quenching material may comprise one or more selected from the group consisting of 4-(dimethylamino)azobenzene-4-carboxylic acid (DABCYL), 4-(dimethylamino)azobenzene sulfonic acid (DABSYL), a blackhole quencher (BHQ), a blackberry quencher (BBQ), an ECLIPSE quencher, a tide quencher, a carbon nanomaterial, and a manganese dioxide nanomaterial.

In one embodiment, the immunoassay device may further include a light source radiating light to the analysis sample solution accommodated in the reaction space of the substrate; and an image analyzer for receiving fluorescence generated from the switching peptide released from the binding antibody to generate an image thereof, and analyzing the image to analyze an amount of the switching peptide released from the binding antibody.

In one embodiment, a single graphene sheet may serve as the support and the quenching material.

An immunoassay method according to an embodiment of the present disclosure includes: a first step of immobilizing a binding antibody to which a switching peptide is bound on a substrate or immobilizing the binding antibody on the substrate, binding the switching peptide to the binding antibody, and binding a quenching material to the binding antibody or the substrate; a second step of treating the binding antibody with a detection sample solution; and a third step of irradiating light to a detection sample solution after the treatment, and quantitatively analyzing a target antigen in the detection sample solution through fluorescence generated from a fluorescent label of the switching peptide released from the binding antibody, wherein the switching peptide comprise a peptide compound having an amino acid sequence capable of selectively and reversibly binding to a fragment antigen-binding (Fab) region of the binding antibody, and a fluorescent label that is bound to the peptide compound and emits fluorescence.

An immunoassay method according to another embodiment of the present disclosure includes: a first step of immobilizing a binding antibody to which a switching peptide is bound on a support or immobilizing the binding antibody on the support, binding the switching peptide to the binding antibody, and binding a quenching material to the binding antibody or the support; a second step of injecting a support to which the binding antibody is bound and a detection sample solution into a reaction space of the substrate; and a third step of irradiating light to the detection sample solution and quantitatively analyzing a target antigen in the detection sample solution through fluorescence generated from a fluorescent label of a switching peptide released from the binding antibody, wherein the switching peptide comprise a peptide compound having an amino acid sequence capable of selectively and reversibly binding to a fragment antigen-binding (Fab) region of the binding antibody, and a fluorescent label that is bound to the peptide compound and emits fluorescence.

In one embodiment, when the target antigen is present in the detection sample solution in the second step, if the target antigen is bound to the binding antibody, the switching peptide may be quantitatively released from the binding antibody depending on an amount of the target antigen that has reacted with the binding antibody.

In one embodiment, fluorescence generated from the fluorescent label of the switching peptide bound to the binding antibody may be quenched by the quenching material, and fluorescence generated from the fluorescent label of the switching peptide released from the binding antibody may be emitted to the outside.

### [Advantageous Effects]

According to a switching peptide of the present disclosure, an immunoassay device and an immunoassay method using the same, the switching peptide bound to the binding antibody may be quantitatively released from the binding antibody depending on the amount of the target antigen that has reacted with the binding antibody, and the fluorescent label of the switching peptide bound to the binding antibody does not emit fluorescence to the outside by the quenching material, whereas the fluorescent label of the switching peptide released from the binding antibody generates fluorescence to the outside. Thus, quantitative analysis of the target antigen in the detection sample may be performed in one step without a washing process, such as removing unreacted antigens, which is generally required in conventional immunoassay methods.

### [Description of Drawings]

FIG. 1 is a diagram for explaining a switching peptide according to an embodiment of the present disclosure.
FIG. 2 is a diagram for explaining a binding antibody.
FIGS. 3A and 3B are diagrams for explaining embodiments of an immunoassay device according to the present disclosure, and FIG. 3C is a diagram for explaining a method for quantitatively analyzing a target antigen in the immunoassay devices illustrated in FIGS. 3A and 3B.
FIG. 4A is diagrams illustrating a method for confirming a specific interaction between L1 and H2 peptides and a specific interaction between L2 and H1 peptides and the measurement results thereof, and FIG. 4B is diagrams for explaining a specific interaction between L1 and H2 peptides and a specific interaction between L2 and H1 peptides analyzed using PyMOL software.
FIGS. 5A to 5C are graphs obtained by analyzing quenching phenomenon of an antibody complex bound to a switching peptide according to the present disclosure.
FIG. 6 is graphs illustrating a fluorescence intensity measured at a bottom of a microplate after reacting lgGs from human, rabbit, goat, and mouse, which are isolated and immobilized on the microplate, respectively, and other proteins (human hepatitis B antigen, C-reactive protein, protein-A, BSA) with fluorescently labeled four types of peptide compounds (H1, H2, L1, L2) synthesized in Example 1.
FIG. 7A is a schematic diagram illustrating states before and after treatment of the antibody to which the four types of peptides of Example 1 were bound, respectively, with papain, which is a protease, and FIG. 7B is graphs illustrating a fluorescence intensity measured at a bottom (B) of the microplate and in a solution (S) after the papain treatment.
FIG. 8A is a schematic diagram for explaining an immunoassay method using fluorescently labeled peptide compounds of Example 1, and FIGS. 8B and 8C are graphs illustrating a fluorescence intensity in a solution and at a bottom of the microplate depending on a concentration of the antigen measured by the immunoassay method illustrated in FIG. 8A.
FIG. 9 is a graph illustrating a fluorescence intensity measured after reacting an anti-HBsAG antibody to which an H2 switching peptide of Example 2 and a Tide-Quencher 2 (TQ2) quenching material are bound, with HBsAg, InfA, InfB (Florida), Inf B (Tokio), and PBS sample solutions, respectively.
FIG. 10 is a graph illustrating a fluorescence intensity measured after reacting the anti-HBsAG antibody to which the H2 switching peptide of Example 2 and the Tide-Quencher 2 (TQ2) quenching material are bound, with detection sample solutions each containing various concentrations of HBsAg antigens.
FIG. 11 is a graph illustrating a fluorescence intensity measured after binding the anti-HBsAG antibody to which the H2 switching peptide of Example 2 and the Tide-Quencher 2 (TQ2) quenching material are bound, to protein A beads and reacting it with detection sample solutions containing various concentrations of the HBsAg antigens.
FIG. 12 is a graph illustrating a fluorescence intensity measured after reacting the anti-HBsAG antibody to which one of the L1, H1, L2 and H2 switching peptides of Example 2 and the TQ2 quenching material is bound, with HRP, CRP, HBsAg, and hCG sample solutions, respectively.
FIG. 13 is graphs illustrating a fluorescence intensity measured after binding an anti-HBsAg antibody to which an L1 switching peptide labeled with a TAMRA fluorescent label is bound, to a graphene quenching material and reacting it with an HBsAg sample solution.
FIG. 14 is a graph illustrating a fluorescence intensity measured after binding an anti-inf A antibody to which an L1 switching peptide labeled with a TAMRA fluorescent label is bound, to a graphene quenching material and reacting it with an Influenza A sample solution.
FIG. 15 is a graph illustrating a fluorescence intensity measured after binding an anti-SARS-CoV-2 antibody to which an L1 switching peptide labeled with a TAMRA fluorescent label is bound, to a graphene quenching material and reacting it with a SARS-CoV-2 sample solution.
FIGS. 16A to 16D are graphs illustrating a fluorescence intensity measured after reacting anti-Inf antibodies to which four types of switching peptides labeled with FAM or TAMRA fluorescent labels are bound, with Inf-A and Inf-B sample solutions.
FIGS. 17A to 17C illustrate the results obtained by measuring a binding affinity (K_{D}) of target antigens (Inf-A and Inf-B) and selected switching peptides (L1 peptide and H2 peptide) to binding antibodies.
FIGS. 18A to 18C illustrate the experimental results for confirming selective detection of the binding antibody bound to the switching peptide according to the present disclosure against Inf-A.
FIGS. 19A to 19C illustrate the experimental results for confirming selective detection of the binding antibody bound to the switching peptide according to the present disclosure against Inf-B.
FIGS. 20A to 20D illustrate the results of detecting Inf-A and Inf-B with an one-step immunoassay device according to the present disclosure and commercially available test kits.

### [Best Mode for Invention]

Hereinafter, exemplary embodiments of the present disclosure will be described in detail with reference to the accompanying drawings. The present disclosure may be variously modified and have several exemplary embodiments. Therefore, specific exemplary embodiments of the present disclosure will be illustrated in the accompanying drawings and be described in detail in the specification. However, it is to be understood that the present disclosure is not limited to a specific disclosed form, but includes all modifications, equivalents, and substitutions without departing from the scope and sprit of the present disclosure. Similar reference numerals have been used for similar components in describing each drawing. In the accompanying drawings, dimensions of the structures have been enlarged as compared with the actual dimensions for clarity of the present disclosure.

Terms as used herein used only in order to describe specific exemplary embodiments rather than limiting the present disclosure. Singular forms include plural forms unless the context clearly indicates otherwise. It should be understood that term "include" or "has" as used herein specify the presence of features, numerals, steps, operations, components described herein, or combinations thereof, but does not preclude the presence or addition of one or more other features, numerals, steps, operations, components, or combinations thereof.

Unless being defined otherwise, it is to be understood that all the terms as used herein including technical and scientific terms have the same meanings as those that are generally understood by one of ordinary skill in the art to which the present disclosure pertains. Terms generally used and defined by a dictionary should be interpreted as having the same meanings as meanings within a context of the related art and should not be interpreted as having ideal or excessively formal meanings unless being clearly defined otherwise in the present specification.

### <Switching peptide>

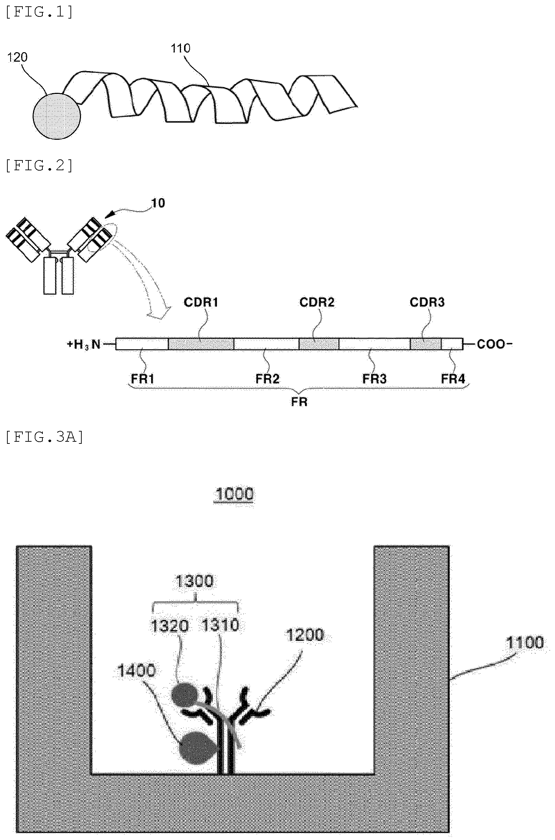

FIG. 1 is a diagram for explaining a switching peptide according to an embodiment of the present disclosure, and FIG. 2 is a diagram for explaining a binding antibody.

Referring to FIGS. 1 and 2, a switching peptide 100 according to an embodiment of the present disclosure may comprise a peptide compound 110 capable of reversibly binding to a binding antibody 10, and a fluorescent label 120 bound to the peptide compound 110. The switching peptide 100 may be reversibly bound to the binding antibody 10 that specifically bound to a target antigen, and thus may be quantitatively released from the binding antibody 10 when the target antigen is specifically bound to the binding antibody. As a result, the electrochemical characteristics of a test sample including the target antigen may be changed by a redox reaction mediated by the fluorescent label 120 of the switching peptide 100 released from the binding antibody 10.

The binding antibody 10 is a compound capable of inducing an immunological effector mechanism by binding to or reacting with a specific antigenic determinant such as an epitope of the target antigen. The binding antibody may be monospecific or polyspecific.

In one embodiment, the binding antibody 10 may comprise an antibody, an antibody derivative, or a fragment thereof. The binding antibody 10 may be an intact immunoglobulin molecule or, alternatively, may comprise components of intact antibodies such as Fab, Fab', F(ab')₂, Fc, F (v), N-glycan structure, paratope, etc., or at least some of the components.

In one embodiment, the binding antibody may be a human antibody, a non-human antibody, or a humanized non-human antibody.

The human antibody may comprise antibodies produced by humans, or synthetic antibodies having amino acid sequences synthesized using any technique. This definition of the human antibody specifically excludes humanized antibodies comprising non-human antigen-binding residues. The human antibody may be produced using a variety of techniques known in the art, including phage-display libraries. The non-human antibody may be an antibody obtained from sources of various species, for example, rodents, rabbits, cattle, sheep, pigs, dogs, non-human mammals, or birds. A "humanized" form of the non-human antibody may be a chimeric antibody that contains minimal sequence derived from non-human immunoglobulin. In one embodiment, the humanized antibody may be a human immunoglobulin (recipient antibody) in which residues from a hypervariable region of the recipient are replaced with the hypervariable region of the aforementioned non-human antibody (donor antibody) having a desired specificity, affinity and/or capacity. In some cases, framework residues of a human immunoglobulin may be replaced by corresponding non-human residues. In addition, the humanized antibody may comprise residues not found in either the recipient antibody or the donor antibody. These modifications may be made to further refine antibody performance, such as binding affinity.

In one embodiment, the binding antibody 10 may be an antibody molecule, an immunoglobulin molecule, a fragment thereof, or an aggregate of one or more of them. The binding antibody 10 may have a unique structure that enables specific binding to the target antigen, and each binding antibody 10 may comprise two light chains of identical structure and two heavy chains of identical structure. The heavy chain and the light chain may comprise a variable region and a constant region, respectively. The variable regions of the heavy chain and the light chain may be combined to form an antigen binding site. In the binding antibody 10, a portion to which a target antigen is bound by a protease such as papain may be separated into an antibody binding fragment (Fab) region and a fragment crystallizable (Fc) region, which is a crystallized supporting pillar portion, where the antigen binding site may be included in the antibody binding fragment (Fab) region of the antibody.

Meanwhile, each of the variable regions of the heavy and light chains may have a structure in which three complementarity-determining regions (CDRs) that are hypervariable regions (HVRs), and four framework regions (FRs) structurally supporting the complementarity-determining regions are alternately arranged, respectively. The complementarity-determining regions have different amino acid sequences for each antibody, and thus may be specifically bound to each antigen, whereas the framework regions have conserved amino acid sequences according to subfamilies of organisms, so antibodies of organisms belonging to the same subfamilies have the same or similar amino acid sequences.

For the convenience of description below, in the variable region of the light chain of the binding antibody 10, the four framework regions arranged sequentially apart from the N-terminus, are referred to as first to fourth light chain variable framework regions (VL-FR1, VL-FR2, VL-FR3, and VL-FR4), respectively, and the three complementarity determining regions, which are located between the first to fourth light chain variable framework regions (VL-FR1, VL-FR2, VL-FR3, and VL-FR4), respectively, and sequentially disposed from the N-terminus, are referred to as first to third light chain variable complementarity determining regions (VL-CDR1, VL-CDR2, VL-CDR3), respectively. In addition, in the variable region of the heavy chain of the binding antibody 10, the four framework regions arranged sequentially apart from the N-terminus are referred to as first to fourth heavy chain variable framework regions (VH-FR1, VH-FR2, VH-FR3, and VH-FR4), respectively, and the three complementarity-determining regions, which are located between first to fourth heavy chain variable framework regions (VH-FR1, VH-FR2, VH-FR3, and VH-FR4), respectively, and sequentially disposed from the N-terminus, are referred to as first to third heavy chain variable complementarity-determining regions (VH-CDR1, VH-CDR2, VH-CDR3), respectively.

The peptide compound 110 may have an amino acid sequence capable of selectively and reversibly binding to the fragment antigen-binding (Fab) region of the binding antibody 10.

In one embodiment, the peptide compound 110 may have an amino acid sequence capable of specifically and reversibly binding to one or more of first to fourth framework regions (FR1, FR2, FR3, and FR4) of a light chain or heavy chain of the binding antibody 10. For example, the peptide compound 110 may comprise an amino acid sequence having homology to one or more of the first to fourth framework regions (FR1, FR2, FR3 and FR4) of the light chain or heavy chain of the binding antibody 10. In this case, the peptide compound 110 may be reversibly bound to one or more of the first to fourth framework regions (FR1, FR2, FR3, and FR4) of the light chain or heavy chain. Accordingly, when the target antigen is bound to the binding antibody 10, the peptide compound 10 bound to the binding antibody 10 may be quantitatively released from the binding antibody 10 depending on the amount of antigen bound to the binding antibody 10.

In one embodiment, the peptide compound 110 may comprise one or more selected from a first peptide compound L1 having a first amino acid sequence having homology to an amino acid sequence of the second light chain variable framework region (VL-FR2), a second peptide compound L2 having a second amino acid sequence having homology to an amino acid sequence of the third or fourth light chain variable framework region (VL-FR3, VL-FR4), a third peptide compound H1 having a third amino acid sequence having homology to an amino acid sequence of the second heavy chain variable framework region (VH-FR2), and a fourth peptide compound H2 having a fourth amino acid sequence having homology to an amino acid sequence of the third or fourth heavy chain variable framework region (VH-FR3, VH-FR4).

In the binding antibody 10, the second light chain variable framework region (VL-FR2) and the third or fourth heavy chain variable framework region (VH-FR3, VH-FR4) are located adjacent to each other and interact with each other, and the third or fourth light chain variable framework region (VL-FR3, VL-FR4) and the second heavy chain variable framework region (VH-FR2) are located adjacent to each other and interact with each other. Thus, the first peptide compound L1 having a first amino acid sequence having homology to the second light chain variable framework region (VL-FR2) of the binding antibody 10 may selectively interact with the third or fourth heavy chain variable framework region (VH-FR3, VH-FR4) of the binding antibody 10, the second peptide compound L2 having a second amino acid sequence having homology to the third or fourth light chain variable framework region (VL-FR3, VL-FR4) of the binding antibody 10 may selectively interact with the second heavy chain variable framework region (VH-FR2) of the binding antibody 10, the third peptide compound H1 having a third amino acid sequence having homology to the second heavy chain variable framework region (VH-FR2) of the binding antibody 10 may selectively interact with the third or fourth light chain variable framework region (VL-FR3, VL-FR4) of the binding antibody 10, and the fourth peptide compound H2 having a fourth amino acid sequence having homology to the third or fourth heavy chain variable framework region (VH-FR3, VH-FR4) of the binding antibody 10 may selectively interact with the second light chain variable framework region (VL-FR2) of the binding antibody 10.

In one embodiment, when the binding antibody 10 is derived from an organism belonging to subfamilies of animals including humans, the peptide compound 110 may comprise about 10 to 25 amino acids and may have a molecular weight of about 1,600 to 3,000 Da. For example, the peptide compound 110 may comprise about 14 to 20 amino acids and may have a molecular weight of about 1,650 to 2, 500 Da. In one embodiment, the first to fourth peptide compounds (L1, L2, H1, H2) may comprise amino acid sequences as shown in Table 1 below.

**[Table 1]**

| Switching peptide | Amino acid sequence | Molecular weight | Gibbs free energy (Kcal/mol) |
|---|---|---|---|
| H1 | SYWIH WVKQR PGQGL EWIGE | 2469.7 | △ G=-17. 91 |
| H2 | VYYCA REPTG TGIYF DVWGK | 2325.6 | △ G=-17.19 |
| L1 | TYLEW YPQKP GQSPK LLIYK | 2452.8 | △ G=-15.37 |
| L2 | VYYCF QGSHV PFTK | 1675.9 | △ G=-13. 05 |

Meanwhile, since the framework regions (FR1, FR2, FR3, FR4) of the light and heavy chains of the antibodies have conserved amino acid sequences according to subfamilies of organisms, the peptide compound 110 may be commonly applied to antibodies derived from different species of organisms belonging to the same subfamilies. Therefore, even when the peptide compound 110 is synthesized from antibodies of goat, mouse, rabbit, etc., it can exhibit the same actions and effects as described above for antibodies of human. The fluorescent label 120 may be bound to the peptide compound 110 and emit fluorescence. The fluorescent label 120 is not particularly limited as long as it can be bound to the peptide compound 110 and can emit fluorescence. For example, the fluorescent label 120 may comprise one or more selected form the group consisting of rhodamine and its derivatives, fluorescein and its derivatives, coumarin and its derivatives, acridine and its derivatives, pyrene and its derivatives, erythrosine and its derivatives, eosin and its derivatives, 4-acetamido-4'-isothiocyanatostilbene-2,2'disulfonic acid, fluorescein isothiocyanate (FITC), oregon green, alex fluoro, carboxyfluorescein (FAM), 6-carboxy-4',5'-dichloro-2',7'-dimethoxyfluorescein (JOE), carboxy-X-rhodamine (ROX), 6-carboxy-2',4,4',5',7,7'-hexachlorofluorescein (HEX), Texas Red (sulforhodamine 101 acid chloride), 6-carboxy-2',4,7',7-tetrachlorofluorescein (TET), tetramethylrhodamine-isothiocyanate (TRITC), carboxytetramethylrhodamine (TAMRA), cyanine-based dyes, and thiadicarbocyanine dyes.

### <Immunoassay device>

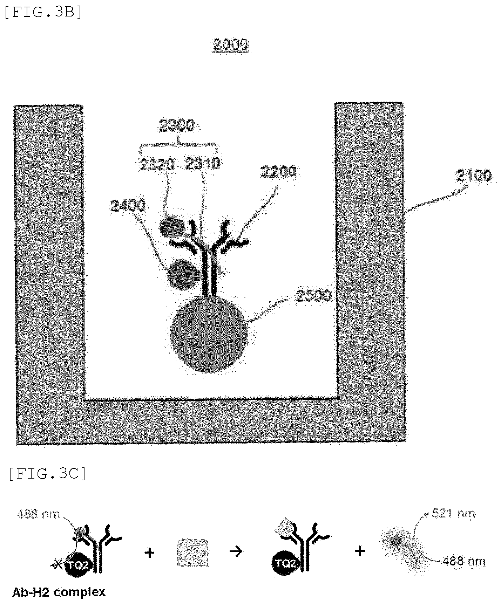

FIGS. 3A and 3B are diagrams for explaining embodiments of an immunoassay device according to the present disclosure, and FIG. 3C is a diagram for explaining a method for quantitatively analyzing a target antigen in the immunoassay devices illustrated in FIGS. 3A and 3B.

Referring to FIGS. 3A and 3C, an immunoassay device 1000 according to an embodiment of the present disclosure may include : a substrate 1100 having a reaction space capable of accommodating a detection sample solution to be analyzed, a binding antibody 1200 located inside the reaction space and immobilized on the substrate 1100, a switching peptide 1300 reversibly bound to a Fab region of the binding antibody 1200, and a quenching material 1400 disposed adjacent to the fluorescent labeling material 1320 of the switching peptide 1300 to quench fluorescence from the fluorescent label 1320.

The structure or shape of the substrate 1100 is not particularly limited as long as it has a reaction space capable of accommodating the detection sample solution. However, the substrate 1100 may be formed of a transparent material capable of transmitting light, for example, a transparent polymer material, a glass material, a metal oxide material, etc.

The binding antibody 1200 is substantially the same as the binding antibody 10 described with reference to FIG. 2. Therefore, an overlapped description thereof will be omitted. The binding antibody 1200 may be immobilized on the substrate 1100 by directly binding to the surface of the substrate 1100 or by binding to the surface of the substrate 1100 through a separate linker compound.

The switching peptide 1300 may comprise a peptide compound 1310 and a fluorescent label 1320. Since the switching peptide 1300 is substantially the same as the switching peptide 100 described with reference to FIG. 1. Therefore, an overlapped description thereof will be omitted.

When the switching peptide 1300 is bound to the binding antibody 1200, the quenching material 1400 may be disposed adjacent to the fluorescent label 1320 to absorb fluorescence energy from the fluorescent label 1320 to quench fluorescence from the fluorescent label 1320.

Even in a negative specimen without the binding antibody 1200, the fluorescent label 1320 is quenched by the quenching material 1400, so a fluorescence signal may not be generated from the detection antibody before and after specimen treatment. Therefore, in the above method, even if the immobilized antibody does not bind to the switching peptide 1300, a uniform immunoassay may be performed in a solution.

In addition, the material of the quenching material 1400 is not particularly limited as long as it can quench fluorescence from the fluorescent label 1320. In one embodiment, the quenching material 140 may comprise one or more selected from the group consisting of 4-(dimethylamino)azobenzene-4-carboxylic acid (DABCYL), 4-(dimethylamino)azobenzene sulfonic acid (DABSYL), a blackhole quencher (BHQ), a blackberry quencher (BBQ), an ECLIPSE quencher, and a tide quencher.

In another embodiment, the quenching material 1400 may comprise a carbon nanomaterial or a manganese dioxide nanomaterial. The carbon nanomaterial may comprise one or more selected from the group consisting of graphene and its derivatives, graphene oxide (NGO) and its derivatives, reduced graphene oxide and its derivatives, and graphene oxide nanocolloid (GON). The nanomaterial may be in the shape of a sheet or a particle. The sheet may be composed of a single layer or a plurality of layers, and the sheet may exist in various shapes including a flat surface or a curved surface. In addition, the shape of particles may include various shapes such as spherical, elliptical, rod, and polygonal shapes.

The quenching material 1400 may absorb fluorescence energy of light or wavelength generated from the fluorescent label 1320 by interaction with the fluorescent label 1320 to quench the fluorescence from the fluorescent label 1320.

In one embodiment, the quenching material 1400 may be disposed adjacent to the fluorescent label 1320 by being bound to the binding antibody 1200. For example, the quenching material 1400 may be bound to the Fc region of the binding antibody 1200. In another embodiment, the quenching material 1400 may be disposed adjacent to the fluorescent label 1320 by being bound to the substrate 1100.

According to the immunoassay device of the present disclosure, as illustrated in FIG. 3C, when a detection sample solution containing a target antigen that specifically reacts with the binding antibody 1200 is injected into the reaction space of the substrate 1100, the target antigen reacts with the binding antibody 1200, and thus the switching peptide 1300 may be quantitatively released from the binding antibody 1200 depending on the amount of the target antigen that reacts with the binding antibody 1200. In addition, the fluorescent labeling material 13200 of the switching peptide 1300 released from the binding antibody 1200 moves away from the quenching material 1400, and fluorescence generated therefrom may be emitted to the outside without being quenched. In the present disclosure, quantitative analysis of the target antigen contained in the detection sample solution may be performed by quantitatively analyzing the switching peptide 1300 released from the binding antibody 1200 through such fluorescence. In this case, since a washing process such as removal of unreacted target antigen is not required, quantitative analysis of the target antigen may be performed in one step after reacting the detection sample solution with the binding antibody.

Meanwhile, although not illustrated in the drawing, in order to generate fluorescence from the fluorescent label 1320 and quantitatively analyze the switching peptide 1300 released from the binding antibody 1200 through this fluorescence, the immunoassay device 1000 may further include the light source and image analysis device.

The light source may irradiate light to an analysis sample solution accommodated in the reaction space of the substrate 1100, and the fluorescent label 1320 of the switching peptide 1300 may generate fluorescence by absorbing light energy irradiated from the light source. In one embodiment, the light source may generate short-wavelength light in the visible light or ultraviolet region and irradiate the analysis sample solution accommodated in the reaction space of the substrate 1100.

The image analysis device may receive fluorescence generated from the switching peptide 1300 released from the binding antibody 1200 to generate an image thereof, and analyze the image to analyze the amount of the switching peptide 1300 released from the binding antibody 1200. For example, the image analysis device may include an image generator for imaging a fluorescence signal, an image processor for processing the image generated by the image generator, and an image analyzer for analyzing the image processed by the image processor.

Meanwhile, referring to FIGS. 3B and 3C, an immunoassay device 2000 according to another embodiment of the present disclosure includes a substrate 2100, a binding antibody 2200, a switching peptide 2300, a quenching material 2400, and a support 2500.

The immunoassay device 2000 of this embodiment is substantially the same as or similar to the immunoassay device 1000 described with reference to FIG. 3A except that the binding antibody 2200 is immobilized on the support 2500 instead of the substrate 2100. Therefore, an overlapped description thereof will be omitted below.

The support 2500 may have a particle shape made of an inorganic or organic material, and the binding antibody 2200 may be bound to the support 2500. In addition, the quenching material 2400 may be bound to the binding antibody 2200 or the support 2500 and disposed adjacent to the fluorescent label 2320 of the switching peptide 2300 bound to the binding antibody 2200.

Meanwhile, FIG. 3B illustrates the support 2500 and the quenching material 2400 as independent components different from each other. However, in one embodiment of the present disclosure, the quenching material is applied to the support 2500, so that the support 2500 and the quenching material 2400 may have the same configuration, and in this case, for example, a carbon material such as graphene may be used as the support 2500.

According to the immunoassay device of this embodiment, since the binding antibody 2200 may be bound to the support 2500 instead of the substrate 2100 to move in the solution, in addition to the advantages of the immunoassay device illustrated in FIG. 3A, a reaction rate between the binding antibody 2300 and the target antigen in the detection sample solution may be further improved.

### <Immunoassay method>

Hereinafter, an immunoassay method according to an embodiment of the present disclosure using the immunoassay device illustrated in FIG. 3A or the immunoassay device illustrated in FIG. 3B will be described in detail.

Referring to FIGS. 3A and 3C, an immunoassay method according to an embodiment of the present disclosure includes: a first step of immobilizing a binding antibody to which a switching peptide is bound on a substrate or immobilizing the binding antibody on the substrate, binding the switching peptide to the binding antibody, and binding a quenching material to the binding antibody or the substrate; a second step of treating the bound antibody with a detection sample solution; and a third step of irradiating light to the detection sample solution after the treatment, and quantitatively analyzing a target antigen in the detection sample solution through fluorescence generated from a fluorescent label of a switching peptide released from the binding antibody.

In the first step, the switching peptide may comprise a peptide compound having an amino acid sequence capable of selectively and reversibly binding to the fragment antigen-binding (Fab) region of the binding antibody, and a chemical label that is bound to the peptide compound and capable of being oxidized and reduced in the detection sample solution. In one embodiment, as the switching peptide and binding antibody, the switching peptide 100 and the binding antibody 10 described with reference to FIGS. 1 and 2 are used. Therefore, an overlapped description thereof will be omitted.

In one embodiment, in the case of the binding antibody and the switching peptide, the binding antibody may be first immobilized on the substrate and then the switching peptide may be bound to the immobilized binding antibody. Alternatively, the switching peptide may be bound to the binding antibody and then the binding antibody may be immobilized on the substrate.

A method of immobilizing the binding antibody on the substrate is not particularly limited. For example, the binding antibody may be directly bound to the substrate, or may be bound to the substrate through a linker compound and immobilized thereto.

As described above, the switching peptide may be selectively and reversibly bound to the fragment antigen-binding (Fab) region of the binding antibody.

The quenching material may be bound to the Fc region of the binding antibody or to a surface within the reaction space of the substrate. Here, the quenching material may be bound to the binding antibody or the substrate so as to be positioned adjacent to the fluorescent label of the switching peptide bound to the binding antibody.

In the second step, the binding antibody may be reacted with the target antigen in the detection sample solution by injecting the detection sample solution into the reaction space of the substrate.

In one embodiment, when there is a target antigen that specifically reacts with the binding antibody in the detection sample solution, if the detection sample solution is injected into the reaction space, the target antigen may be bound to the binding antibody, and in this case, the switching peptide may be quantitatively released from the binding antibody depending on the amount of the target antigen reacted with the binding antibody.

In the third step, the detection sample solution may be injected into the reaction space of the substrate, and light may be irradiated to the detection sample solution after a predetermined period of time has elapsed. In this case, the fluorescence generated from the fluorescent label of the switching peptide bound to the binding antibody is quenched by the quenching material disposed adjacent thereto and is not emitted to the outside, whereas the fluorescence generated from the fluorescent label of the switching peptide released from the binding antibody may be spatially spaced apart from the quenching material and may be emitted to the outside without being quenched.

In addition, after generating an image of the fluorescence generated from the fluorescent label of the switching peptide released from the binding antibody using the image analysis device, the image is analyzed to perform quantitative analysis on the switching peptide released from the binding antibody, and from the results thereof, the amount of the target antigen contained in the detection sample solution may be quantitatively calculated. Quantitative analysis of the amount of the target antigen as described above may be possible because the switching peptide bound to the binding antibody is quantitatively released from the binding antibody depending on the amount of the target antigen that reacts with the binding antibody.

Meanwhile, referring to FIGS. 3B and 3C, an immunoassay method according to another embodiment of the present disclosure includes: a first step of immobilizing a binding antibody to which a switching peptide is bound on a support or immobilizing the binding antibody on the support, binding the switching peptide to the binding antibody, and binding a quenching material to the binding antibody or the support; a second step of injecting a support to which the binding antibody is bound and a detection sample solution into a reaction space of the substrate; and a third step of irradiating light to a detection sample solution after the treatment, and quantitatively analyzing a target antigen in the detection sample solution through fluorescence generated from a fluorescent label of the switching peptide released from the binding antibody.

In the first step, the binding antibody may be bound to the support rather than the substrate, and in the second step, the binding antibody bound to the support may flow inside the detection sample solution. Meanwhile, the remaining steps except for this are substantially the same as the immunoassay method using the immunoassay device illustrated in FIG. 3A. Therefore, an overlapped description thereof will be omitted.

According to a switching peptide of the present disclosure, an immunoassay device and an immunoassay method using the same, the switching peptide bound to the binding antibody may be quantitatively released from the binding antibody depending on the amount of the target antigen that has reacted with the binding antibody, and the fluorescent label of the switching peptide bound to the binding antibody does not emit fluorescence to the outside by the quenching material, whereas the fluorescent label of the switching peptide released from the binding antibody generates fluorescence to the outside. Thus, quantitative analysis of the target antigen in the detection sample may be performed in one step without a washing process, such as removing unreacted antigens, which is generally required in conventional immunoassay methods.

Hereinafter, specific embodiments of the present disclosure will be described in detail. However, the following examples are merely some embodiments of the present disclosure, and the scope of the present disclosure is not limited to the following examples.

### [Mode for Invention]

### [Example 1]

Four types of peptides (H1, H2, L1, L2) as shown in Table 1 were synthesized. Specifically, the H1 peptide was synthesized to have an amino acid sequence having homology to the FR2 region of the heavy chain, the H2 peptide was synthesized to have an amino acid sequence having homology to the FR3 or FR4 region of the heavy chain, the L1 peptide was synthesized to have an amino acid sequence having homology to the FR2 region of the light chain, and the L2 peptide was synthesized to have an amino acid sequence having homology to the FR3 or FR4 region of the light chain.

A change in Gibbs free energy (ΔG) was -17.91 kcal/mol for the H1 peptide compound, -17.19 kcal/mol for the H2 peptide, 15.37 kcal/mol for the L1 peptide, and -13.05 kcal/mol for the L2 peptide.

### [Experimental Example 1]

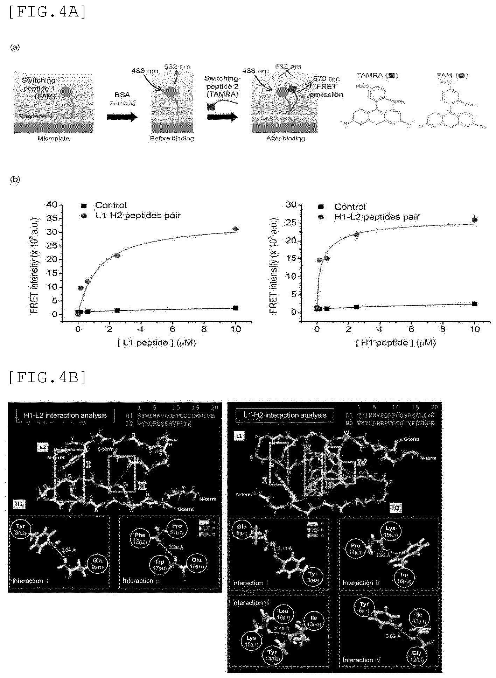

FIG. 4A is diagrams illustrating a method for confirming a specific interaction between L1 and H2 peptides and a specific interaction between L2 and H1 peptides and the measurement results thereof, and FIG. 4B is diagrams for explaining a specific interaction between L1 and H2 peptides and a specific interaction between L2 and H1 peptides analyzed using PyMOL software. Here, at the ends of the L1 and L2 peptides, a first fluorescent label, FAM (λₑₓ = 488 nm, λₑₘ = 532 nm) was modified, and at the ends of the H1 and H2 peptides, a second fluorescent label, TAMRA (λₑₓ = 532 nm, λₑₘ = 570 nm) was modified. Meanwhile, the first peptide (L1 or L2) was immobilized on a parylene-H-coated microplate through a covalent bond between a formyl group of parylene-H and a primary amine group of a lysine residue in the peptide, and a second peptide (H2 or H1) was incubated with the immobilized first peptide (L1 or L2) to allow specific interactions between them. On the other hand, it can be seen that a specific interaction between an immobilized first peptide (L1 or L2) and a second peptide (H2 or H1) incubated therewith may be determined by whether the FRET effect with green fluorescence emission from TAMRA (λₑₘ = 570 nm) modified to the second peptide was observed at the bottom of the microplate, and when a specific interaction between the first and second peptides occurs, the FRET effect was observed at the bottom of the microplate.

Referring to FIG. 4A, the interaction between the L1 and H2 peptides was observed by changing the concentration of the L1 peptide, and the FRET intensity increased with increasing the concentration of the L1 peptide. These results indicate that a specific interaction between the L1 and H2 peptides has occurred.

Also, the interaction between the H1 and L2 peptides was observed by changing the concentration of the H1 peptide, and the FRET intensity was observed to increase with increasing the concentration of the H1 peptide. These results also indicate that a specific interaction between the H1 and L2 peptides has occurred.

From the above results, it can be seen that the L1 peptide is selectively and reversibly bound to the FR3 or FR4 region of the heavy chain (corresponding to the H2 peptide), the L2 peptide is selectively and reversibly bound to the FR 2 region of the heavy chain (corresponding to the H1 peptide), the H1 peptide is selectively and reversibly bound to the FR3 or FR4 region of the light chain (corresponding to the L2 peptide), and the H2 peptide is selectively and reversibly bound to the FR2 region of the light chain (corresponding to the L1 peptide).

Referring to FIG. 4B, as a result of analyzing the hydrogen bond between H1-L2 peptides and the hydrogen bond between H2-L1 peptides through PyMOL, in the case of between H1-L2 peptides, (1) a hydrogen bond with a length of 3.34 Å between glutamine (residue number 9 of the H1 peptide) and tyrosine (residue number 3 of the L2 peptide) and (2) a hydrogen bond with a length of 3.39 Å between a carbonyl group of a peptide bond from the L2 peptide and an electron deficient hydrogen of the peptide bond from the H1 peptide were found, and in the case of between H2-L1 peptides, four hydrogen bonds with a length of 2.49 Å to 3.93 Å were found (1) between amino acids (interaction I), (2) between peptide bonds (interactions II, III), and (3) between amino acids and peptide bonds (interaction IV) . The number of hydrogen bonds indicates that the interaction between the H1-L2 peptides is stronger than that between the H2-L1 peptides, whereas similar values of changes in Gibbs free energy suggest similar binding affinities of peptide compounds to IgG.

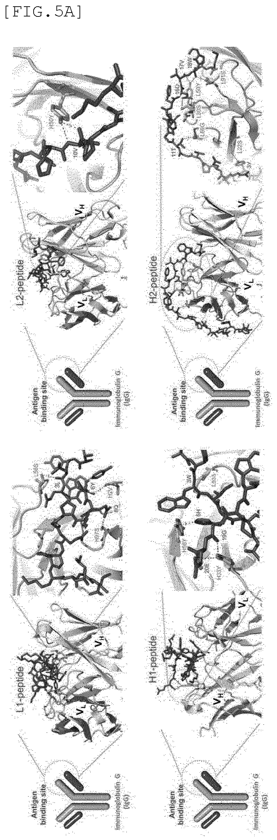

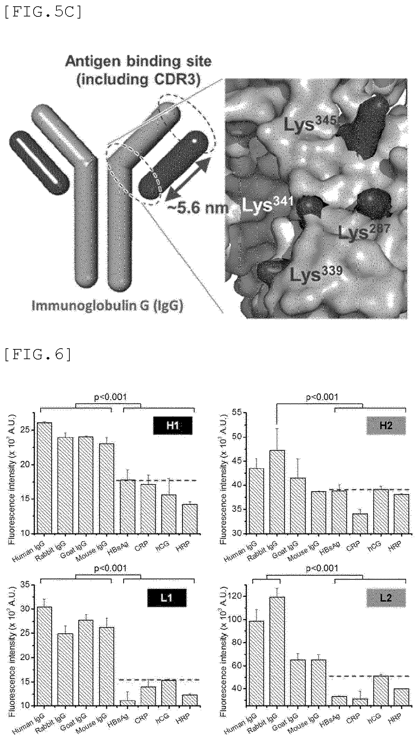

FIGS. 5A to 5C are graphs obtained by analyzing quenching phenomenon of an antibody complex bound to a switching peptide according to the present disclosure.

FIG. 5A is a computer simulation image for finding a binding site of the switching peptide in IgG. Referring to FIG. 5A, the L1 and L2 peptides may be bound to the H2 and H1 regions of heavy chain of the IgG (V_{H}), respectively, while the H1 and H2 peptides may be bound to the L2 and L1 regions of the light chain of IgG (V_{L}), respectively. Four switching peptides were docked to an antigen-binding site of IgG (mouseFab fragment, PDB ID = 1DQJ) using computerized docking simulation using the CABS-dock web server. To analyze the binding of the switching peptide, a binding pocket of IgG, an amino acid sequence of a CDR region of a model antibody were taken from reference: CDR1 (amino acid numbers: 31-35), CDR2 (amino acid numbers: 50-65), and CDR3 (amino acid numbers: 95-102) in V_{H}, and CDR1 (amino acid numbers: 24-34), CDR2 (amino acid numbers: 50-56), and CDR3 (amino acid numbers: 89-97) in V_{L}.

Amino acids were analyzed for the interaction between the switching peptide and the antibody region through computer simulation, which is the same as described with reference to FIGS. 1A and 1B. In addition, the average distance from amino acid for the interaction between the switching peptide and the antibody region was estimated in the range of 2.5 to 3.5 Å. Interaction parameters between each switching peptide and the Fv-region of IgG are described below as shown in Table 2.

**[Table 2]**

| Peptide name | Amino Acids for Interaction | | Distance (Å) |
|---|---|---|---|
| | Switching peptide | Antibody | |
| H2 peptide | Tyr³ | Ser²² (FR1, V_{L}) | 3.15 |
| | Thr¹¹ | Gly⁶⁶ (FR3. V_{L}) | 3.11 |
| | Asp¹⁶ | Ser³⁰ (CDR1, V_{L}) | 2.61, 3.12 |
| | Val¹⁷ | Tyr⁵⁰ (CDR2, V_{L}) | 3.19 |
| | Trp¹⁸ | Tyr⁵⁰ (CDR2, V_{L}) | 2.76 |
| | | Ser⁹¹ (CDR3, V_{L}) | 2.69 |
| L1 peptide | Leu³ | Ser⁵⁶ (CDR2, V_{L}) | 3.09 |
| | Tyr⁶ | Va¹²(FR1, V_{H}) | 3.01 |
| | Gln⁸ | Ser⁹⁷ (FR3, V_{H}) | 2.09 |
| L2 peptide | Val¹⁰ | Try⁵⁰(FR2, V_{L}) | 2.58, 3.01 |
| H1 peptide | Trp³ | Ser⁹³ (CDR3, V_{L}) | 3.03 |
| | His⁵ | Tyr⁵⁸ (FR3, V_{H}) | 3.45 |
| | Gly¹⁹ | Tyr³³ (FR2, V_{H}) | 3.38 |
| | Glu²⁰ | | 3.55 |

After binding switching peptides labeled with two types of fluorescent labels (FAM, TAMRA) to two types of binding antibodies labeled with quenching materials (TQ2, TQ3), fluorescence quenching efficiency was measured. In general, the labeling of the quenching material is known to occur in the Fc region of an antibody, and a size of an antigen-binding fragment of IgG is known to be within 2.5 nm in diameter at V_{H}. It was confirmed that the locations of the four types of switching peptides in the antigen-binding pocket of the IgG of the present disclosure were not significantly different from those of a quenching label.

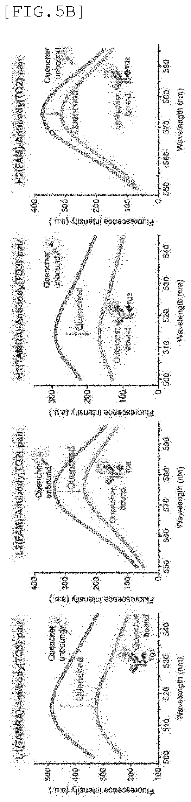

FIG. 5B is graphs illustrating intensity of fluorescence signal for the antibody bound to the switching peptide before and after labeling with a quenching material. As illustrated in FIG. 5B, it can be confirmed that a reduction level of fluorescence signal varies depending on the combination of the fluorescent labels (FAM, TAMRA) of the switching peptide and the quenching materials (TQ2, TQ3) of the binding antibody. As shown in Table 3 below, fluorescence quenching efficiency (E) was 0.33 for TQ2 in the H2 peptide bound to FAM, 0.43 for TQ2 in the L2 peptide bound to FAM, 0.16 for TQ3 in the L1 peptide bound to TAMRA, and 0.28 for TQ3 in the H1 peptide bound to TAMRA. Therefore, it can be confirmed that the L2 peptide bound to FAM has a higher fluorescence quenching efficiency by TQ2 than the H2 peptide bound to FAM, and when bound to TAMRA, the H1 peptide has higher fluorescence quenching efficiency to TQ3 than the L1 peptide.

In addition, Table 3 showed that the distance between the fluorescent label and the quenching material was 55.8Å for TQ2 in the H2 peptide bound to FAM, 52.3 Å for TQ2 in the L2 peptide bound to FAM, 54.5 Å for TQ3 in the L1 peptide bound to TAMRA, and 47.6 Å for TQ3 in the H1 peptide bound to TAMRA.

**[Table 3]**

| Binding form | E | K² | J (M⁻¹cm³) | n | Φ | ε_{Amax} (M⁻¹cm⁻¹) | Ro (Å) | Distance (Å) |
|---|---|---|---|---|---|---|---|---|
| H2 peptide (FAM)-TQ2 | 0.33 | 0.67 | 1.11 × 10¹⁵ | 1.40 | 0.90 | 48 × 10³ | 49.75 | 55.83 |
| L2 peptide (FAM)-TQ2 | 0.43 | 0.67 | 1.16 × 10¹⁵ | 1.40 | 0.90 | 48 × 10³ | 50.13 | 52.58 |
| L1 peptide (TAMRA)-TQ2 | 0.16 | 0.67 | 3.15 × 10¹⁵ | 1.40 | 0.90 | 90 × 10³ | 41.06 | 54.46 |
| H1 peptide (TAMRA)-TQ2 | 0.28 | 0.67 | 3.02 × 10¹⁵ | 1.40 | 0.90 | 90 × 10³ | 40.78 | 47.58 |

FIG. 6 is graphs illustrating a fluorescence intensity measured at a bottom of a microplate after reacting lgGs from human, rabbit, goat, and mouse, which are isolated and immobilized on the microplate, respectively, and other proteins (human hepatitis B antigen, C-reactive protein, protein-A, BSA) with fluorescently labeled four types of peptide compounds (H1, H2, L1, L2) synthesized in Example 1.

Referring to FIG. 6, only IgGs from human, rabbit, goat, and mouse showed significantly high fluorescence intensities, and other antigenic proteins showed baseline level of fluorescence. These results show that the peptides of Example 1 are bound selectively to the antibody.

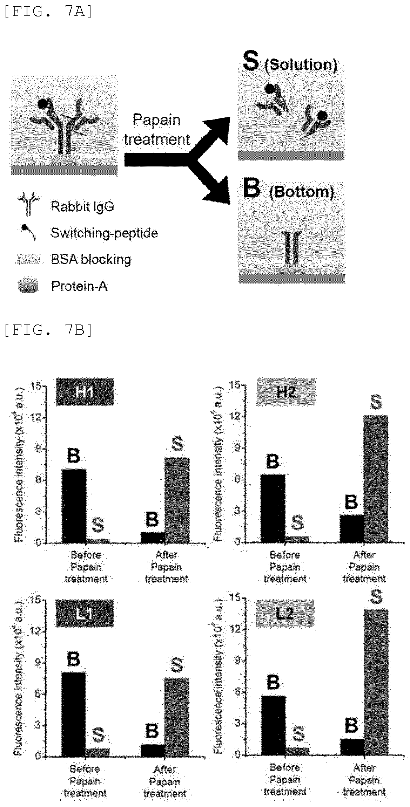

FIG. 7A is a schematic diagram illustrating states before and after treatment of the antibody to which the four types of peptides of Example 1 were bound, respectively, with papain, which is a protease, and FIG. 7B is graphs illustrating a fluorescence intensity measured at a bottom (B) of the microplate and in a solution (S) after the papain treatment. In the case of this experiment, after immobilization of the antibodies (rabbit anti-HRP antibodies) on the microplate, binding between the antibody and the switching peptide was induced by treatment with a fluorescently labeled switching peptide, the unbound switching peptide was removed, and then the Fab region was hydrolyzed from the immobilized antibodies by adding papain, which is a protease.

Referring to FIG. 7A, the fluorescence at the bottom (B) of the microplate is expected to decrease after the papain reaction because the fluorescently labeled switching peptides are bound to the Fab region of antibodies, and the fluorescence in the solution is expected to increase because the Fab region to which the switching peptide is bound is hydrolyzed and dissolved into the solution. This prediction is proven to be true from the graphs of FIG. 7B.

Referring to FIG. 7B, the fluorescence at the bottom (B) of the microplate decreased and the fluorescence in solution (S) increased, after papain treatment compared to before papain treatment in all four types of switching peptides (H1, H2, L1, L2) . Specifically, for the fluorescence intensity at the bottom of the microplate, it was evaluated that the H1 peptide decreased by 84.3%, the H2 peptide decreased by 58.7%, the L1 peptide decreased by 84.8%, and the L2 peptide decreased by 72.5%, respectively. Also, for the fluorescence intensity in the solution, it was evaluated that the H1 peptide increased by 2,184%, the H2 peptide increased by 2,356%, the L1 peptide increased by 1,032%, and the L2 peptide increased by 2,452%, respectively.

From the above results, it can be seen that the four types of switching peptides of Example 1 are bound to the Fab regions of the antibody.

Table 4 below shows the results of evaluating the binding properties of the four types of peptide compounds (H1, H2, L1, L2) of Example 1 to antibodies from different animals (rabbit, mouse, and goat). These results are fluorescence intensity values measured after binding fluorescently labeled switching peptides to antibodies immobilized on a microplate, removing unreacted switching peptides, and then reacting with target antigens (CRP for rabbit IgG, hCG for mouse IgG, HRP for rabbit IgG, HBsAg for goat IgG), respectively.

**[Table 4]**

| Pept ide | Antibody (r=rabbit , g-goat, m-mouse) | In solution (S) | | | Bottom (B) of microplate | | |
|---|---|---|---|---|---|---|---|
| | | Before antigen binding | After antigen binding | Change (%) | Before antigen binding | After antigen binding | Change (%) |
| H1 | Anti-HRP antibody( r) | 2596(±704) | 21965(±707 8) | +746 | 11840(±290 ) | 1451(±103) | -87.7 |
| H1 | Anti-HBsA 9 antibody( g) | 2242(±91) | 11453(±829 ) | 1410 | 11225(±305 ) | 4618(±71) | -58.9 |
| H1 | Anti-hCG antibody( m) | 1281(±63) | 3921(±120) | +206 | 5646(±20) | 1940(±74) | -65.6 |
| H1 | Anti-CRP antibody( r) | 2242(±91) | 5659(±100) | 1152 | 4912(±1999 ) | 3595(±30) | -26.8 |
| H2 | Anti-HRP antibody( r) | 2272(±668) | 18128(±145 6) | +698 | 122230(±30 9) | 1806(±290) | -85.2 |
| H2 | Anti-HBsA g antibody( g) | 1784(±113) | 9136(±835) | 1412 | 29367(±766 ) | 18773(±117 6) | -36.1 |
| H2 | Anti-hCG antibody( m) | 1654(±54) | 8545(±1291 ) | +416 | 23320(±326 0) | 23320(±326 0) | -88.5 |
| H2 | Anti-CRP antibody( r) | 2099(±120) | 16311(±165 ) | +677 | 22205(±732 ) | 22205(±732 ) | -39.5 |
| L1 | Anti-HRP antibody( r) | 2687(±711) | 12280(±314 1) | +357 | 15608(±638 ) | 1990(±251) | -87.3 |
| L1 | Anti-HBsA g antibody( g) | 2779(±293) | 20501(±228 5) | +638 | 11062(±462 6) | 5388(±581) | -51.3 |
| L1 | Anti-hCG antibody( m) | 1248(±76) | 3580(±416) | +187 | 4630(±676) | 1777(±44) | -61.6 |
| L1 | Anti-CRP antibody( r) | 741(±197) | 6505(±61) | +778 | 6719(±132) | 3456(±178) | -48.6 |
| L2 | Anti-HRP antibody( r) | 2315(±754) | 16135(±110 7) | +597 | 12584(±210 ) | 3208(±117) | -74.5 |
| L2 | Anti-HBsA 9 antibody( g) | 2279(±635) | 15359(±405 0) | +574 | 28002(±316 ) | 9856(±1150 ) | -64.8 |
| L2 | Anti-hCG antibody( m) | 1692(±126) | 9402(±656) | +456 | 33156(±498 0) | 3072(±184) | -90.7 |
| L2 | Anti-CRP antibody( r) | 3274(±353) | 32187(±138 35) | +883 | 20394(±54) | 11236(±72) | -44.9 |

Referring to Table 4, it was measured that the fluorescence intensity of each immobilized antibody (IgG) decreased at the bottom of the plate and increased in the solution after treatment with the corresponding antigen. Specifically, in the case of anti-HRP antibodies from rabbits, for H1, L1, H2 and L2 peptides, the fluorescence intensity at the bottom of the plate decreased by 12.3%, 12.7%, 14.8%, and 25.5%, respectively, and the fluorescence intensity in the solution increased by 746 0, 357%, 698%, and 597%, respectively. In the case of anti-HBsAg antibodies from goats, for H1, L1, H2, and L2 peptides, the fluorescence intensity at the bottom of the plate decreased by 41.1%, 48.7%, 63.9%, and 35.2%, respectively, and the fluorescence intensity in the solution increased by 410%, 638%, 412%, and 574%, respectively. These results show that the four types of peptide compounds synthesized in Example 1 are not only capable of binding to the immobilized antibody (IgG) regardless of the species of the source animal, but are also quantitatively released from the antibody when the antigen was bound to the binding pocket of the antibody due to the reversible binding of each of the four types of peptide compounds of Example 1 to the antibody.

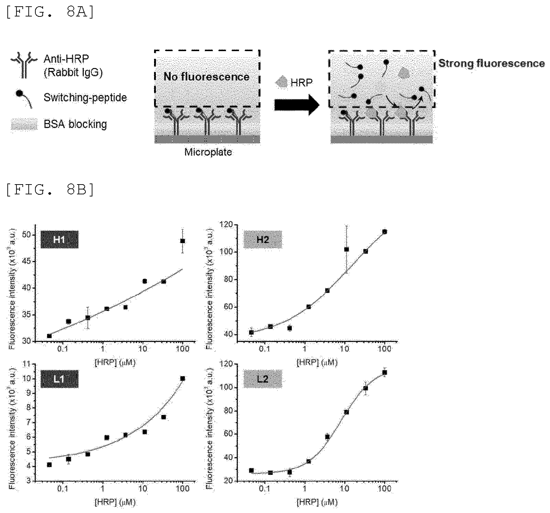

FIG. 8A is a schematic diagram for explaining an immunoassay method using fluorescently labeled peptide compounds of Example 1, and FIGS. 8B and 8C are graphs illustrating the fluorescence intensity in a solution and at a bottom of the microplate depending on a concentration of the antigen measured by the immunoassay method illustrated in FIG. 8A. In this experiment, an anti-HRP antibody from a rabbit immobilized on a microplate was used as the binding antibody and HRP was used as the antigen.

Referring to FIGS. 8A to 8C, as a result of performing immunoassay while varying the concentration of antigen HRP from 0.3 to 100 µM, for the four types of switching peptides (H1, H2, L1, L2), an increase in the fluorescence intensity in the solution was observed depending on the concentration of the antigen (HRP).

Equilibrium dissociation constants of the H1, H2, L1, and L2 peptides were estimated to be 1.65, 3.11, 3.29, and 1.48 µM, and the difference in the dissociation constant for each switching peptide means that each switching peptide has a different detection area for each antigen. For the HRP antigen, the H1 peptide reacted most linearly in the HRP concentration region. These results show that the switching peptide was released from the immobilized antibody according to the bound amount of the antigen, and quantitative analysis of the antigen was possible based on the fluorescence intensity in the solution in one-step immunoassay using the switching peptide.

Meanwhile, FIG. 8C illustrated that the fluorescence intensity at the bottom of the microplate decreased depending on the concentration of the antigen in all four types of switching peptides. These results show that the switching peptide was quantitatively released from the antibody depending on the amount of the antigen, and quantitative analysis of the antigen was possible through a change in the fluorescence intensity in the solution as well as at the bottom of the microplate.

### [Example 2]

The switching peptides shown in Table 5 below were synthesized by binding fluorescein amidite (FAM) fluorescent labels to the ends of each of the L1, L2, H1, and H2 peptide compounds.

**[Table 5]**

| Switching peptide | Amino acid sequence | Molecular weight (Da) |
|---|---|---|
| L1 | FAM-T-Y-L-E-W-Y-P-Q-K-P-G-Q-S-P-K-L-L-I-Y-K | 2663.34 |
| L2 | FAM-V-Y-Y-C-F-Q-G-S-H-V-P-F-T-K | 1753 |
| H1 | FAM-S-Y-W-I-H-W-V-K-Q-R-P-G-Q-G-L-E-W-I-G-E | 2681.02 |
| H2 | FAM-V-Y-Y-C-A-R-E-P-T-G-T-G-I-Y-F-D-V-W-G-K | 2408.02 |

### [Experimental Example 2]

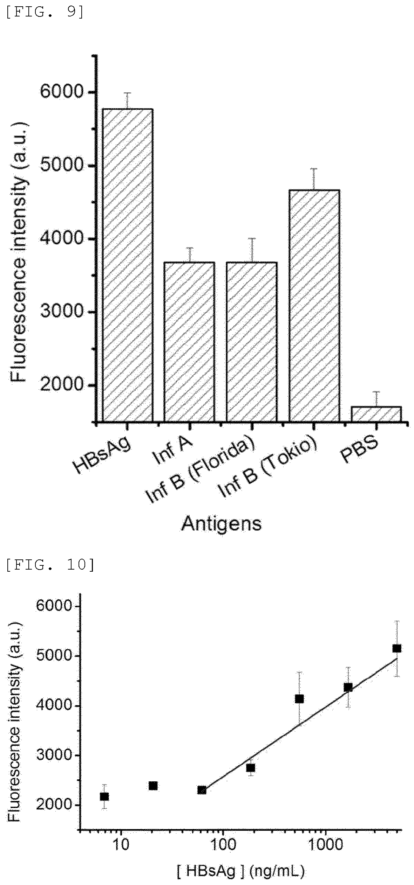

FIG. 9 is a graph illustrating a fluorescence intensity measured after reacting an anti-HBsAG antibody to which an H2 switching peptide of Example 2 and a Tide-Quencher 2 (TQ2) quenching material are bound, with HBsAg, Inf A, Inf B (Florida), Inf B (Tokio), and PBS sample solutions, respectively.

Referring to FIG. 9, a high intensity fluorescence was measured only for the HBsAg antigen, and a relatively low fluorescence intensity was measured for the other antigens.

These results show that the switching peptide was quantitatively released from the binding antibody depending on the amount of the target antigen bound to the binding antibody, and the fluorescent label of the switching peptide released from the binding antibody mainly emitted fluorescence to the outside.

FIG. 10 is a graph illustrating a fluorescence intensity measured after reacting the anti-HBsAG antibody to which the H2 switching peptide of Example 2 and the Tide-Quencher 2 (TQ2) quenching material are bound, with detection sample solutions each containing various concentrations of HBsAg antigens.

Referring to FIG. 10, it was found that the fluorescence intensity increased linearly in proportion to the concentration of HBsAg in the region where the concentration of HBsAg was from about 50 ng/mL to about 500 ng/mL. These results show that the target antigen may be quantitatively analyzed when the H2 switching peptide of Example 2 and the TQ2 quenching material are applied.

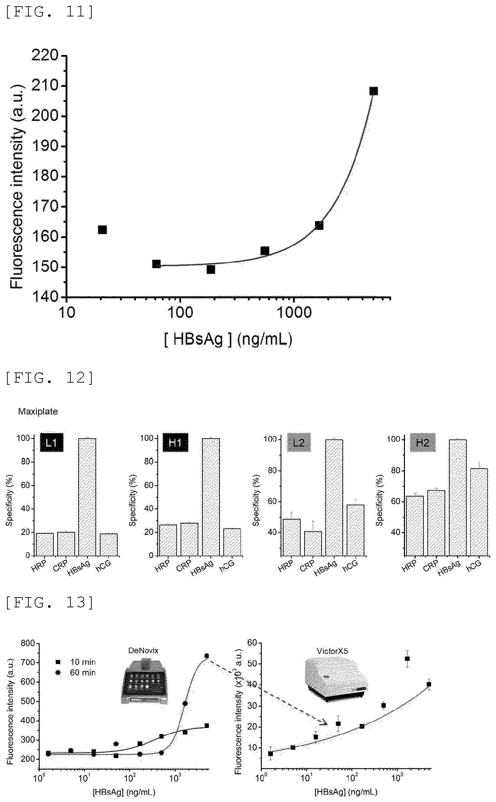

FIG. 11 is a graph illustrating a fluorescence intensity measured after binding the anti-HBsAG antibody to which the H2 switching peptide of Example 2 and the Tide-Quencher 2 (TQ2) quenching material are bound, to protein A beads and reacting it with detection sample solutions containing various concentrations of the HBsAg antigens.

Referring to FIG. 11, it was found that the measured fluorescence intensity increased as the concentration of HBsAg antigens of the detection sample solution increased. These results show that when the H2 switching peptide of Example 2 and the TQ2 quenching material are bound to beads and reacted with a detection sample solution containing the target antigen, the target antigen may be quantitatively analyzed.

FIG. 12 is graphs illustrating a fluorescence intensity measured after reacting the anti-HBsAG antibody to which one of the L1, H1, L2 and H2 switching peptides of Example 2 and the TQ2 quenching material is bound, with HRP, CRP, HBsAg, and hCG sample solutions, respectively.

Referring to FIG. 12, for all of the L1, H1, L2 and H2 switching peptides, a high intensity fluorescence was measured only for the HBsAg antigen, and a relatively low fluorescence intensity was measured for the other antigens. However, it was found that when the H2 switching peptide was applied, the specificity for the antigen was relatively low compared to the case where other switching peptides were applied.

These results show that the switching peptide was quantitatively released from the binding antibody depending on the amount of the target antigen bound to the binding antibody, and the fluorescent label of the switching peptide released from the binding antibody mainly emitted fluorescence to the outside.

FIG. 13 is graphs illustrating a fluorescence intensity measured after binding an anti-HBsAg antibody to which an L1 switching peptide labeled with a TAMRA fluorescent label is bound, to a graphene quenching material and reacting it with an HBsAg sample solution.

Referring to FIG. 13, it was found that the measured fluorescence intensity increased almost linearly as the concentration of the HBsAg antigen contained in the HBsAg sample solution increased. These results show when the binding antibody to which an L1 switching peptide labeled with a TAMRA fluorescent label is bound, is bound to a graphene quenching material and reacted with the detection sample solution, the target antigen in the detection sample solution may be quantitatively analyzed.

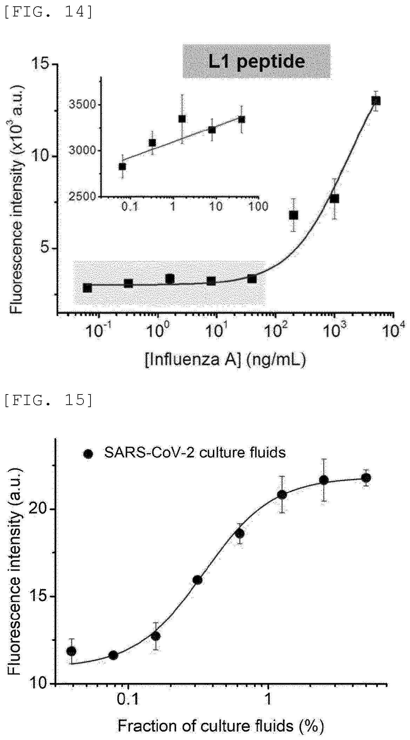

FIG. 14 is a graph illustrating a fluorescence intensity measured after binding an anti-inf A antibody to which an L1 switching peptide labeled with a TAMRA fluorescent label is bound, to a graphene quenching material and reacting it with an Influenza A sample solution.

Referring to FIG. 14, it was found that the measured fluorescence intensity increased almost linearly as the concentration of the Influenza A antigen contained in the Influenza A sample solution increased. These results show when the binding antibody to which an L1 switching peptide labeled with a TAMRA fluorescent label is bound, is bound to a graphene quenching material and reacted with the detection sample solution, the target antigen in the detection sample solution may be quantitatively analyzed.

FIG. 15 is a graph illustrating a fluorescence intensity measured after binding an anti-SARS-CoV-2 antibody to which an L1 switching peptide labeled with a TAMRA fluorescent label is bound, to a graphene quenching material and reacting it with a SARS-CoV-2 sample solution.

Referring to FIG. 15, it was found that the measured fluorescence intensity increased as the concentration of the SARS-CoV-2 antigen contained in the SARS-CoV-2 sample solution increased. These results show when the binding antibody to which an L1 switching peptide labeled with a TAMRA fluorescent label is bound, is bound to a graphene quenching material and reacted with the detection sample solution, the target antigen in the detection sample solution may be quantitatively analyzed.

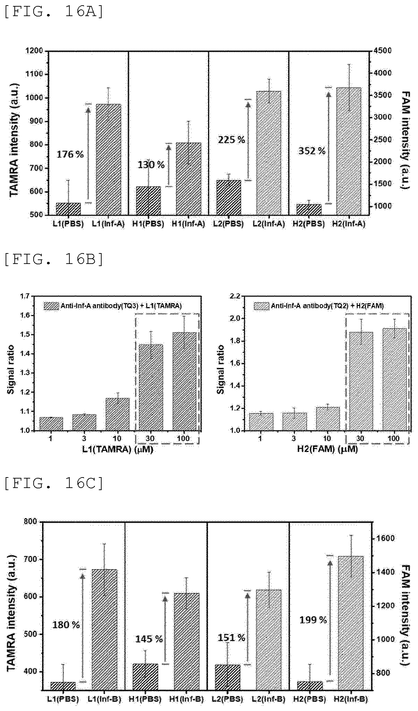

FIGS. 16A to 16D are graphs illustrating a fluorescence intensity measured after reacting anti-Inf antibodies to which four types of switching peptides labeled with FAM or TAMRA fluorescent labels are bound, with Inf-A and Inf-B sample solutions.

Referring to FIG. 16A, it was confirmed that the L1 and H1 peptides labeled with TAMRA showed a relative change in fluorescence signal of 176% and 130%, respectively, and the L2 and H2 peptides labeled with FAM showed a relative change in fluorescence signal of 225% and 352%, respectively. Therefore, the dissociation of the switching peptide from the binding antibody to Inf-A was observed to be the highest in the L1 peptide labeled with TAMRA and the H2 peptide labeled with FAM, and thus, they may be a optimal combination of the switching peptide and the fluorescent label for the one-step immunoassay.

To confirm the optimal concentration of the switching peptide for the one-step immunoassay, antibodies labeled with quenching materials were detected at the concentration of 250 ug/mL using switching peptides in the range of 1 to 100 µM (binding antibody labeled with TQ3 to the L1 peptide labeled with TAMRA, and binding antibody labeled with TQ2 to the H2 peptide labeled with FAM) . From FIG. 16B, it can be confirmed that the fluorescence signal from the binding antibody was measured, and the optimum concentration of the switching peptide was about 30 µM for the binding antibody labeled with TQ2 or TQ3.

In addition, experiments on Inf-B were also conducted. Referring to FIG. 16C, it was confirmed that the L1 and H1 peptides labeled with TAMRA showed a relative change in fluorescence signal of 180% and 145%, respectively, and the L2 and H2 peptides labeled with FAM showed a relative change in fluorescence signal of 151% and 199%, respectively. Therefore, it was found that the dissociation of the switching peptide from the binding antibody to Inf-B is the highest in the H2 peptide labeled with FAM, and among the four types of switching peptides, the L1 peptide labeled with TAMRA and the H2 peptide labeled with FAM are useful.

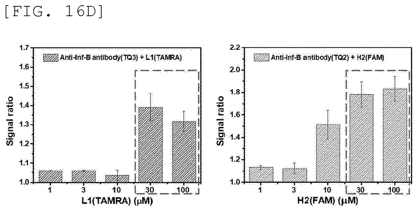

To confirm the optimal concentration of the switching peptide for the one-step immunoassay, antibodies labeled with quenching materials were detected at the concentration of 250 ug/mL using switching peptides in the range of 1 to 100 µM (binding antibody labeled with TQ3 to the L1 peptide labeled with TAMRA, and binding antibody labeled with TQ2 to the H2 peptide labeled with FAM) . From FIG. 16D, it can be confirmed that the fluorescence signal from the binding antibody was measured, and the optimum concentration of the switching peptide was about 30 µM for the binding antibody labeled with TQ2 or TQ3.

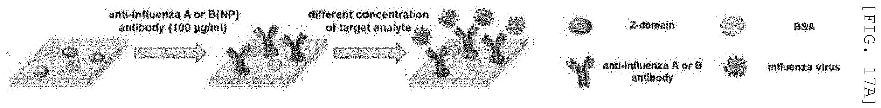

For the one-step immunoassay, when an antigen is detected in a state in which a switching peptide bound to a fluorescent label is bound to a binding antibody, the switching peptide needs to dissociate immediately and quantitatively from the binding antibody. Therefore, the binding affinity of the antigen to the binding antibody needs to be higher than the binding affinity to the switching peptide. Thus, FIGS. 17A to 17C illustrate the results obtained by measuring a binding affinity (K_{D}) of target antigens (Inf-A and Inf-B) and selected switching peptides (L1 peptide and H2 peptide) to binding antibodies through experiments.

Referring to FIG. 17A, anti-Inf-A and anti-Inf-B binding antibodies were immobilized on an Au substrate using an SPR biosensor, and the blank surface of the substrate was blocked with a BSA solution to treat Inf-A and Inf-B.

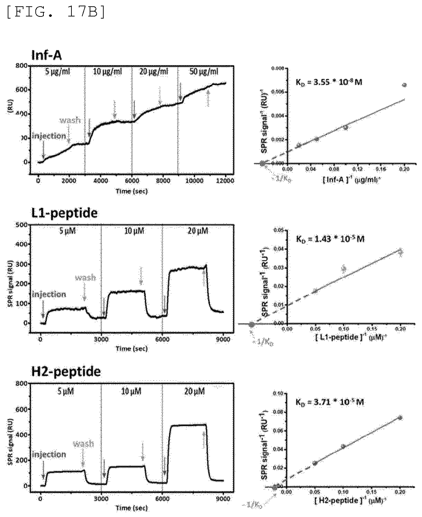

FIG. 17B is graphs obtained by calculating the SPR signal for each sample concentration from a signal difference between a baseline before sample injection (injection and odd-numbered arrows) and a baseline after washing (wash and even-numbered arrows) in the experiment performed with reference to FIG. 17A. Referring to FIG. 17B, it was estimated that the binding affinity (K_{D}) was 3.6 × 10⁻⁸ M for the Inf-A, 1.4 x 10⁻⁵ M for the L1 peptide, and 3.7 × 10⁻⁵ M for the H2 peptide .

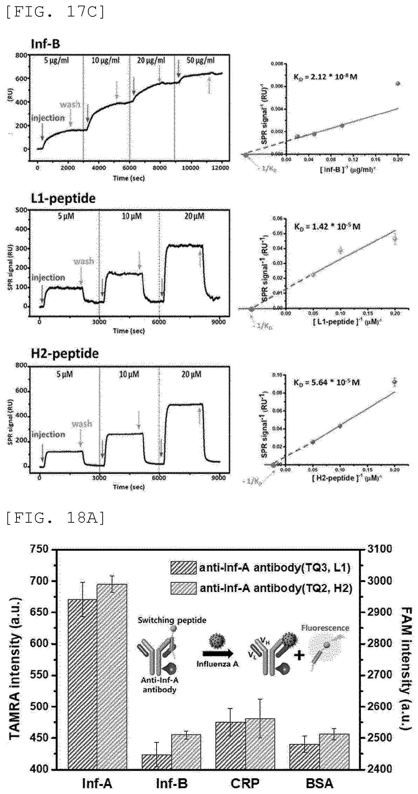

FIG. 17C is graphs illustrating the experimental results for Inf-B. Referring to FIG. 17C, it was estimated that the binding affinity (K_{D}) was 2.1 × 10⁻⁸ M for the Inf-B, 1.4 x 10⁻⁵ M for the L1 peptide, and 5.6 x 10⁻⁵ M for the H2 peptide. Therefore, it can be seen that the binding antibody has much stronger binding to Inf-A and Inf-B than the switching peptide, and the switching peptide dissociates from the IgG binding pocket when Inf-A and Inf-B are bound to the binding antibody. Therefore, it can be confirmed that Inf-A and Inf-B may be detected using the switching peptide and immunoassay device according to the present disclosure.

FIGS. 18A to 18C illustrate the experimental results for confirming selective detection of the binding antibody bound to the switching peptide according to the present disclosure against Inf-A.

Referring to FIG. 18A, it can be seen that from a comparison result obtained by treating the anti-Inf-A antibody bound to the L1 peptide and the anti-Inf-A antibody bound to the H2 peptide with Inf-A, Inf-B, CRP, and BSA, significantly higher fluorescence signal is generated when the Inf-A is treated. Therefore, an antibody bound to the L1 peptide or the H2 peptide may be used for selective detection of Inf-A.

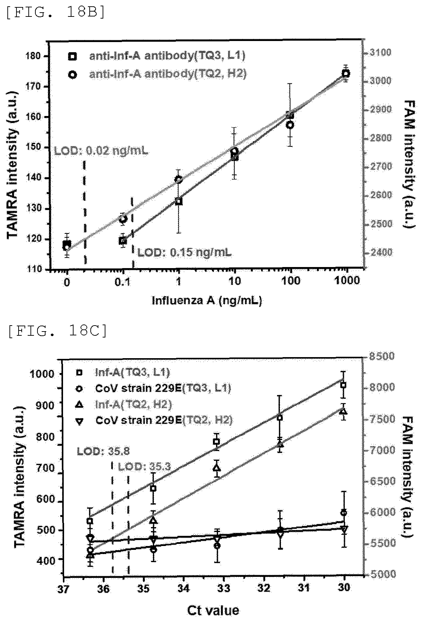

As illustrated in FIG. 18B, as a result of measuring the fluorescence signal according to the concentration of Inf-A for the antibody bound to the L1 peptide and the antibody bound to the H2 peptide in PBS, a limit of detection (LOD) of the anti-Inf-A binding antibody bound to the H2 peptide (quenched with TQ2) was 0.02 ng/mL, and a limit of detection of the anti-Inf-A binding antibody bound to the L1 peptide (quenched with TQ1) was 0.15 ng/mL. Therefore, binding antibodies bound to the two types of peptides may have similar responses upon detection of Inf-A.

FIG. 18C illustrates a result obtained by measuring the number of cycle threshold (Ct) of an anti-Inf-A binding antibody bound to a switching peptide for Inf-A. Referring to FIG. 18C, analysis of actual samples was performed using NATtrol^{™} Influenza A stock from Zeptometriz containing heat-treated Inf-A in the sample matrix, and the actual sample was serially diluted in a control sample matrix (NATtrol^{™} Influenza stock). The fluorescence signal was observed to increase quantitatively over the entire concentration range, and it was estimated that the number of cycle threshold was 35.3 for an anti-Inf-A-binding antibody labeled with FAM bound to the H2 peptide and 35.8 for the anti-Inf-A binding antibody bound to the L1 peptide and labeled with TAMRA. These results show that the two types of binding antibodies may have similar responses to Inf-A detection.

In addition, CoV strain 229E was prepared as a negative control, and analysis was performed using the same two types of binding antibodies, and as a result, it can be confirmed that it is maintained at the baseline. Therefore, an immunoassay based on these two types of binding antibodies may be used to detect Inf-A in actual samples.

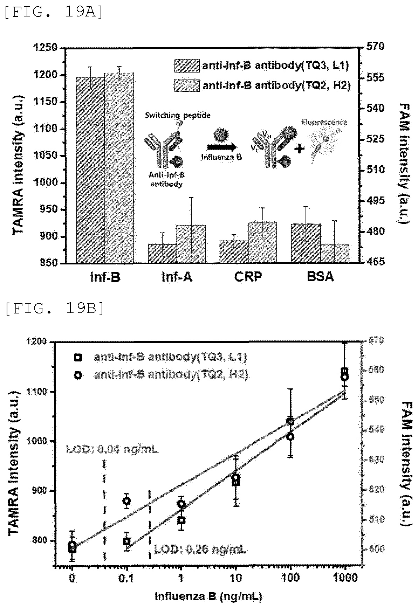

For Inf-B, the results of experiments performed in the same manner as in FIGS. 18A to 18C are shown in FIGS. 19A to 19C.

Referring to FIG. 19A, it can be confirmed that from a comparison result obtained by treating the anti-Inf-B antibody bound to the L1 peptide and the anti-Inf-A antibody bound to the H2 peptide with Inf-A, Inf-B, CRP, and BSA, significantly higher fluorescence signal was generated when the Inf-B is treated. Therefore, an antibody bound to the L1 peptide or the H2 peptide may be used for selective detection of the Inf-B.

As illustrated in FIG. 19B, as a result of measuring the fluorescence signal according to the concentration of Inf-B for the antibody bound to the L1 peptide and the antibody bound to the H2 peptide in PBS, a limit of detection (LOD) of the anti-Inf-B binding antibody bound to the H2 peptide (labeled with FAM) was 0.04 ng/mL, and a limit of detection of the anti-Inf-B binding antibody bound to the L1 peptide (labeled with TAMRA) was 0.26 ng/mL. Therefore, binding antibodies bound to the two types of peptides may have similar responses upon detection of Inf-A.

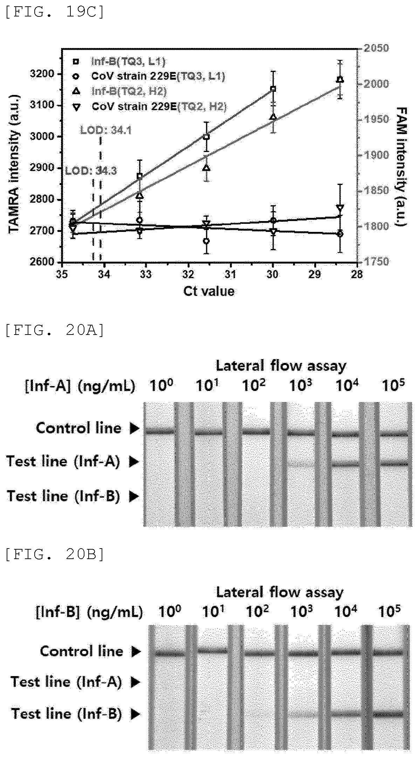

FIG. 19C illustrates a result obtained by measuring the number of cycle threshold (Ct) of an anti-Inf-B binding antibody bound to a switching peptide for Inf-B. As illustrated in FIG. 19C, the fluorescence signal was observed to increase quantitatively over the entire concentration range, and it was estimated that the number of cycle threshold was 34.1 (n = 3) for an anti-Inf-B-binding antibody labeled with FAM bound to the H2 peptide and 34.3 (n = 3) for the anti-Inf-B binding antibody bound to the L1 peptide and labeled with TAMRA. These results show that the two types of binding antibodies may have similar responses to Inf-B detection. In addition, since it can be confirmed that the baseline is maintained in the experiment with CoV strain 229E, which is a negative control, the immunoassay based on the two types of binding antibodies may be used to detect Inf-B in actual samples.

FIGS. 20A to 20D illustrate the results of detecting Inf-A and Inf-B with an one-step immunoassay device according to the present disclosure and commercially available test kits.

Referring to FIG. 20A, lateral flow assay using a Rapid kit from SD Biosensor was performed at a concentration in the range of 1 to 105 ng/mL, and an apparent detection limit was estimated to be less than 105 nm/mL in a test line. Subsequently, as illustrated in FIG. 20B, a fluorescence signal result (lateral flow assay) for an Inf-A standard sample was compared with a result (homogeneous assay) performed using the immunoassay device according to the present disclosure. It was estimated that the detection limit was 546 ng/mL for the lateral flow assay, and 0.02 ng/mL for the immunoassay device according to the present disclosure. Therefore, the immunoassay device using a switching peptide according to the present disclosure may sensitively detect Inf-A in a much wider detection range than conventional rapid tests.

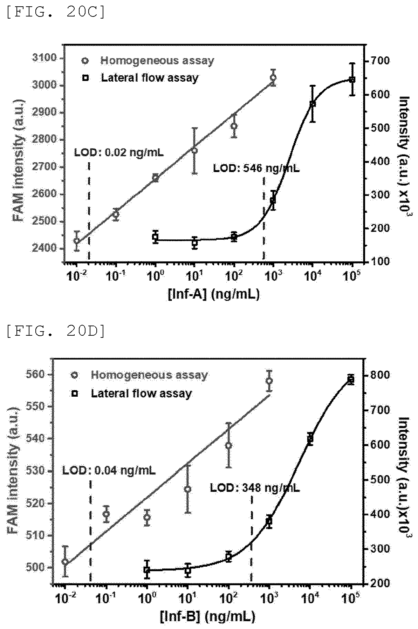

In addition, it was estimated that referring to FIG. 20C, the apparent detection limit in the lateral flow assay using the Rapid kit for Inf-B was about 102 ng/mL in the test line, and as illustrated in FIG. 20D, the detection limit of the lateral flow assay was 348 ng/mL, and the detection limit of the immunoassay device according to the present disclosure was 0.04 ng/mL. Therefore, the immunoassay device using the switching peptide according to the present disclosure may sensitively detect Inf-B in a much wider detection range than conventional rapid tests, and the one-step immunoassay method according to the present disclosure may be used for much more sensitive detection of Inf-A and Inf-B in the entire detection range compared to conventional rapid tests.

Although exemplary embodiments of the present disclosure have been disclosed hereinabove, it may be understood by those skilled in the art that the present disclosure may be variously modified and altered without departing from the scope and spirit of the present disclosure described in the following claims.

## Claims

1. A switching peptide comprising:
a peptide compound capable of reversibly binding to a binding antibody; and
a fluorescent label bound to the peptide compound.

2. The switching peptide of claim 1, wherein the peptide compound is selectively and reversibly bound to a fragment antigen-binding (Fab) region of the binding antibody.

3. The switching peptide of claim 1, wherein the peptide compound has an amino acid sequence capable of specifically and reversibly binding to one or more of first to fourth framework regions (FR1, FR2, FR3, and FR4) of a light chain or heavy chain of the binding antibody.

4. The switching peptide of claim 3, wherein the peptide compound comprises one or more selected from the group consisting of a first peptide compound having a first amino acid sequence having homology to an amino acid sequence of the second light chain variable framework region (VL-FR2); a second peptide compound having a second amino acid sequence having homology to an amino acid sequence of the third or fourth light chain variable framework region (VL-FR3, VL-FR4); a third peptide compound having a third amino acid sequence having homology to an amino acid sequence of the second heavy chain variable framework region (VH-FR2); and a fourth peptide compound having a fourth amino acid sequence having homology to an amino acid sequence of the third or fourth heavy chain variable framework region (VH-FR3, VH-FR4).

5. The switching peptide of claim 4, wherein the peptide compound comprises 14 to 20 amino acids.

6. The switching peptide of claim 5, wherein the peptide compound has a molecular weight of 1,650 to 2,500 Da.

7. The switching peptide of claim 1, wherein the fluorescent label comprises one or more selected form the group consisting of rhodamine and its derivatives, fluorescein and its derivatives, coumarin and its derivatives, acridine and its derivatives, pyrene and its derivatives, erythrosine and its derivatives, eosin and its derivatives, 4-acetamido-4'-isothiocyanatostilbene-2,2'disulfonic acid, fluorescein isothiocyanate (FITC), oregon green, alex fluoro, carboxyfluorescein (FAM), 6-carboxy-4',5'-dichloro-2',7'-dimethoxyfluorescein (JOE), carboxy-X-rhodamine (ROX), 6-carboxy-2',4,4',5',7,7'-hexachlorofluorescein (HEX), Texas Red (sulforhodamine 101 acid chloride), 6-carboxy-2',4,7',7-tetrachlorofluorescein (TET), tetramethylrhodamine-isothiocyanate (TRITC), carboxytetramethylrhodamine (TAMRA), cyanine-based dyes, and thiadicarbocyanine dyes.

8. An immunoassay device comprising:
a substrate having a reaction space capable of accommodating a detection sample solution;
a binding antibody located inside the reaction space and immobilized on the substrate;
a switching peptide comprising a peptide compound reversibly bound to a Fab region of the binding antibody and a fluorescent label bound to the peptide compound; and
a quenching material disposed adjacent to the fluorescent label to quench fluorescence from the fluorescent label.

9. An immunoassay device comprising:
a substrate having a reaction space capable of accommodating a detection sample solution;
a support located inside the reaction space;
a binding antibody bound to the support;
a switching peptide comprising a peptide compound reversibly bound to a Fab region of the binding antibody and a fluorescent label bound to the peptide compound; and
a quenching material disposed adjacent to the fluorescent label to quench fluorescence from the fluorescent label.

10. The immunoassay device of claim 8, wherein the quenching material is bound to the Fc region of the binding antibody or the substrate.

11. The immunoassay device of claim 9, wherein the quenching material is bound to the Fc region of the binding antibody or the support.

12. The immunoassay device of claim 8 or 9, wherein the quenching material comprises one or more selected from the group consisting of 4-(dimethylamino)azobenzene-4-carboxylic acid (DABCYL), 4-(dimethylamino)azobenzene sulfonic acid (DABSYL), a blackhole quencher (BHQ), a blackberry quencher (BBQ), an ECLIPSE quencher, a tide quencher, a carbon nanomaterial, and a manganese dioxide nanomaterial.

13. The immunoassay device of claim 8 or 9, further comprising
a light source radiating light to the analysis sample solution accommodated in the reaction space of the substrate; and
an image analyzer for receiving fluorescence generated from the switching peptide released from the binding antibody to generate an image thereof, and analyzing the image to analyze an amount of the switching peptide released from the binding antibody.

14. The immunoassay device of claim 9, wherein a single graphene sheet serves as the support and the quenching material.

15. An immunoassay method comprising:
a first step of immobilizing a binding antibody to which a switching peptide is bound on a substrate or immobilizing the binding antibody on the substrate, binding the switching peptide to the binding antibody, and binding a quenching material to the binding antibody or the substrate;
a second step of treating the binding antibody with a detection sample solution; and
a third step of irradiating light to a detection sample solution after the treatment, and quantitatively analyzing a target antigen in the detection sample solution through fluorescence generated from a fluorescent label of the switching peptide released from the binding antibody,
wherein the switching peptide comprise a peptide compound having an amino acid sequence capable of selectively and reversibly binding to a fragment antigen-binding (Fab) region of the binding antibody, and a fluorescent label that bound to the peptide compound and emits fluorescence.

16. An immunoassay method comprising:
a first step of immobilizing a binding antibody to which a switching peptide is bound on a support or immobilizing the binding antibody on the support, binding the switching peptide to the binding antibody, and binding a quenching material to the binding antibody or the support;
a second step of injecting a support to which the binding antibody is bound and a detection sample solution into a reaction space of the substrate; and
a third step of irradiating light to the detection sample solution and quantitatively analyzing a target antigen in the detection sample solution through fluorescence generated from a fluorescent label of a switching peptide released from the binding antibody,
wherein the switching peptide comprise a peptide compound having an amino acid sequence capable of selectively and reversibly binding to a fragment antigen-binding (Fab) region of the binding antibody, and a fluorescent label that is bound to the peptide compound and emits fluorescence.

17. The immunoassay method of claim 15 or 16, wherein when the target antigen is present in the detection sample solution in the second step, if the target antigen is bound to the binding antibody, the switching peptide is quantitatively released from the binding antibody depending on an amount of the target antigen that has reacted with the binding antibody.

18. The immunoassay method of claim 17, wherein fluorescence generated from the fluorescent label of the switching peptide bound to the binding antibody is quenched by the quenching material, and
fluorescence generated from the fluorescent label of the switching peptide released from the binding antibody is emitted to the outside.
